# EUROPÄISCHE PATENTSCHRIFT

(11) **EP 0 608 791 B1**
(45) Veröffentlichungstag und Bekanntmachung des Hinweises auf die Patenterteilung: **09.07.1997**
(21) Anmeldenummer: 94100924.3
(22) Anmeldetag: 22.01.1994
(51) Int. Cl.: G01N 33/543, G01N 33/547, G01N 33/551, G01N 33/554

(54) **Immunologischer Test**
Immunoassay
Essai immunologique

(30) Priorität: 26.01.1993 DE 4302012
(43) Veröffentlichungstag der Anmeldung: 03.08.1994
(73) Patentinhaber: SEROSEARCH GmbH - ENTWICKLUNG UND KONZEPTION LABORDIAGNOSTISCHE PRODUKTE BERGHAUSEN, D-76327 Pfinztal (DE)
(72) Erfinder: Naser, Karin, Dipl.-Biol., D-70195 Stuttgart (DE)

(56) Entgegenhaltungen:
- EP-A- 0 331 808
- EP-A- 0 498 658
- DATABASE WPI Week 8917, Derwent Publications Ltd., London, GB; AN 89-125385 & JP-A-01 069 954 (SHINOTEST KENKYUSHO ET AL.) 15. März 1989
- JOURNAL OF CLINICAL MICROBIOLOGY, Bd.14, Nr.5, November 1981, WASHINGTON DC Seiten 486 - 491 DESMONTS ET AL. 'Immunoglobulin M-Immunosorbent Agglutination Assay for Diagnosis of Infectious Diseases: Diagnosis of Acute Congenital and Acquired Toxoplasma Infections'
- THE JOURNAL OF BIOLOGICAL CHEMISTRY, Bd.242, Nr.7, 10. April 1967, BALTIMORE MD Seiten 1651 - 1659 AVRAMEAS ET AL. 'Biologically Active Water-insoluble Protein Polymers'
- I.M. ROITT "Leitfaden der Immunologie", Steinkopf Verlag Darmstadt 1984, Seiten 132-134
- R. KELLER "Immunologie und Immunpathologie", Goerg Thieme Verlag Stuttgart 1987, Seiten 165-168
- E. BENJAMINI u. S. LESKOWITZ "Immunologie", Schwer Verlag Stuttgart 1988, Seiten 102-104

## Beschreibung

Die vorliegende Erfindung betrifft einen neuen immunologischen Test zur qualitativen und quantitativen Erfassung und Bestimmung von antigenspezifischen Immunglobulinen der IgX-Klasse, in der X die Bedeutung von G, M, A, D oder E hat, bzw. von Antikörpern und von Antigenen auf Basis einer Antigen-Antikörper Reaktion, insbesondere einer Agglutinationsreaktion, sowie geeignete Vorrichtungen zur Durchführung eines solchen Tests und deren Verwendung in der Diagnostik bei Säugetieren und beim Menschen.

In seiner allgemeinsten Darstellung läßt sich der erfindungsgemäße Test und die damit verbundenen Vorrichtungen vorteilhafterweise zur Suche, Erfassung und Bestimmung von Immunglobulinen bzw. Antikörpern verwenden, die durch Kontakt mit einem Immunogen oder Antigen, welches in einem Säugetier oder in einem Menschen oder außerhalb davon vorkommen kann, gebildet werden, aber auch zur Suche, Erfassung und Bestimmung eines Immunogens oder Antigens (Antigen-Screening).

Es ist bekannt, daß die Immunoglobuline aus fünf verschiedenen Isotypen oder Klassen bestehen, die sich nicht nur in ihrer antigenen Reaktivität (serologisch), in ihrem Kohlenhydratanteil (chemisch) und in ihrer Form und Größe von einander unterscheiden, sondern auch in ihren immunchemischen und biologischen Eigenschaften und dem Ort ihrer Synthese. Entsprechend dieser Kriterien werden die Immunglobuline in die fünf Klassen IgG, IgM, IgA, IgD und IgE unterteilt.

Prinzipiell bestehen alle Immunglobulinmoleküle hinsichtlich ihrer Struktur aus mindestens zwei schweren, langen H-Ketten und zwei leichten, kurzen L-Ketten, die durch eine Anzahl an Disulfidbrücken miteinander verbunden sind. Bei einigen Immunglobulinen, z.B. IgM, liegen diese Verhältnisse mehrfach vor. Ferner ist allen Immunglobulinen gemeinsam, daß ihre schweren und leichten Ketten eine variable und eine konstante Region aufweisen.

Die einzelnen Immunglobulinklassen unterscheiden sich u.a. in ihren Molekulargewichten, in ihrer Halbwertszeit, in ihrer Konzentration im Serum, ihrer Planzentagängigkeit, ihrer relativen Agglutinierungsfähigkeit, ihrer antiviralen Aktivität oder in ihrer antibakteriellen Aktivität gegenüber Gramnegativen Bakterien erheblich.

Hinsichtlich ihrer Molekulargewichte können die folgenden Werte (in Dalton, d) annähernd zugrundegelegt werden: IgG = 150.000d, IgM = 900.000d, IgA = 160.000d (als Monomer), IgE = 200.000d, IgD = 180.000d. Für die Halbwertszeiten sind die folgenden Werte (in Tagen) bekannt: IgG = 23, IgM = 5, IgA = 5.5, IgE = 2.0, IgD = 2.8. Die Konzentration im Serum ist nicht nur zwischen den einzelnen Klassen der Immunglobuline sehr verschieden, sondern hängt auch entscheidend vom Entwicklungsstadium des Individuums ab. Beim Menschen können für Neugeborene (N) und für Erwachsene (A) die folgenden Serumkonzentrationen (mg/ml) angenommen werden: IgG = 12 (N,A), IgM = 0.1 (N)/1.0 (A), IgA = 0.05 (N)/1.8 (A), IgE = 0 (N)/0.0003 (A), IgD = 0 (N)/0.03 (A). Als einziges Immunglobulin kann nur der IgG-Isotyp die uteroplazentare Schranke passieren und dem Fetus eine gewisse Immunität gegenüber Antigenen verleihen, wobei die Plazentarpassage nur durch den Fc-Anteil des IgG-Isotyps vermittelt wird und die F(ab')2- oder die Fab-Fragmente davon allein die uteroplazentare Schranke nicht passieren können.

Hinsichtlich der relativen Agglutinationsfähigkeit ist das IgM der wirksamste Isotyp, gefolgt vom IgA und anschließend vom IgG. Für das IgE und das IgD spielt diese Eigenschaft eine mehr untergeordnete Rolle. Die Bedeutung der antiviralen und/oder antibakteriellen Aktivität liegt besonders bei den Isotypen IgG, IgM und IgA, wobei für das pentamere und mit multipler Valenz ausgestattete IgM die antivirale Aktivität nur geringe Bedeutung hat. Andererseits befähigt die pentamere Form und die multiple Valenz das IgM ganz besonders dazu, beispielsweise zelluläre Antigene, die relativ weit voneinander entfernte oder sich wiederholende antigene Determinanten (Epitope) auf ihrer Oberfläche exprimieren, multipel zu binden bzw. zu agglutinieren.

Da die Immunglobuline spezifisch Antigene binden können, soll im Folgenden der für Immunglobuline synonym gebrauchte Begriff Antikörper verwendet werden.

Die für alle Antikörper bekannte charakteristische Spezifität hinsichtlich ihrer antigenbindenden Eigenschaften befindet sich am N-terminalen Ende der entsprechenden vier Polypeptidketten der variablen Regionen, d.h. an den Enden der Fab-Anteile der jeweiligen Antikörper, wohingegen die kostanten Regionen (Fc-Anteile) eine Anzahl bekannter biologischer Eigenschaften und Wirkungen mediieren (wie z.B. die oben erwähnte Plazentarpassage beim IgG). Als antigenbindende Antikörperanteile (Fragmente), die selbst ihre antigenspezifischen Eigenschaften beibehalten, sind beispielsweise die Fab-, die Fab'- und die F(ab')2 - Fragmente zu nennen. Als nicht antigenspezifische Fragmente, die insbesondere für die biologischen Eigenschaften der Antikörper verantwortlich sind, können beispielsweise die Fc- und die Fc'- Fragmente genannt werden.

Für die Bedeutung der einzelnen Antikörper hinsichtlich des erfindungsgemäßen Tests sind die jeweils oben charakterisierten Antikörper nicht nur isoliert, sondern auch im Zusammenhang mit ihren biologischen Wirkungen und Eigenschaften zu betrachten. Demgemäß können beispielsweise in Bezug auf die oben dargestellten Konzentrationsunterschiede der Antikörper im Serum die Antikörper des Isotyps IgG die quantitativ wichtigste Klasse darstellen. Sie stellt beispielsweise mit ungefähr 12 mg/ml im Serum des Menschen etwa 15% des Gesamt-Serumproteins dar und liegt mehr oder weniger gleich im intravaskulären und extravaskulären Raum verteilt vor. Mit Ausnahme der IgG3-Unterklasse, deren Halbwertszeit bei ungefähr 7 Tagen liegt, beträgt die Halbwertszeit der IgG-Isotypen (IgG1, lgG2, lgG4) ca. 23 Tage. IgG Antikörper sind wirksame agglutinierende Antikörper und können insbesondere die Agglutination von partikulären (unlöslichen) Antigenen verursachen. Bekannterweise vermittelt IgG die Neutralisation von beispielsweise Toxinen, Giften und Viren und immobilisiert, d.h. verklumpt, Einzeller und Mikroorganismen, wie z.B. Bakterien, Pilze, Flagellaten oder Sporozoen (beispielsweise Toxoplasmen). Die Antikörper der IgM-Klasse finden sich vorwiegend im intravaskulären Raum und liegen beim erwachsenen Menschen in einer Konzentration von ca. 1 mg/ml im Serum vor. Da IgM vornehmlich unmittelbar dann synthetisiert wird, wenn der juvenile oder adulte Organismus eines Säugetiers bzw. Menschen einem Antigenstimulus ausgesetzt wird (z.B. nach Immunisierung oder durch infektiöse Agentien), zeigen erhöhte IgM-Spiegel oder IgM-Titer in der Regel eine kurz vorher eingetretene Infektion oder eine erfolgte Antigen Exposition an. Somit, da IgM Antikörper schon in frühen Infektionsstadien auftreten, folgt, daß die Identifizierung von IgM ein wesentlicher Bestimmungsfaktor für das frühe Erkennen einer Infektionskrankheit oder irgendeiner Antigen Exposition ist. Bekannt ist außerdem, daß Antikörper der IgM-Klasse wirksame agglutinierende Antikörper sind. Zu den IgM-Antikörpern gehören auch die natürlich vorkommenden Isohämagglutinine, die gegen die Erythrozyten-Antigene der ABO-Blutgruppen gerichtet sind.

Das meiste IgA kommt in Sekreten (z.B. Tränenflüssigkeit, Speichel, Schweiß, Schleim, Kolostrum) vor und wird in den entsprechenden Orten synthetisiert (Tränendrüsen, Speicheldrüsen, Gastrointestinaltrakt, Mukosa, Milchdrüsen der laktierenden Brust). Insofern wird dem IgA eine besondere Bedeutung bei der primären immunologischen Abwehr gegen lokale Infektionen zugesprochen, beispielsweise bei Infektionen des Respirations- und Gastrointestinaltrakts oder allgemein bei lokalen Infektionsereignissen. Sekretorisches IgA ist ein wirksamer antiviraler Antikörper und besitzt bakterizide Aktivität insbesondere gegen Gram-negative Bakterien und ist ein wirksamer agglutinierender Antikörper.

Das ebenfalls agglutinierende IgE spielt gleichsam eine bedeutende Rolle bei der Abwehr von Mikroorganismen und anderen Einzellern, aber auch von mehrzelligen Parasiten (z.B. Spulwürmer).Von Bedeutung ist IgE auch für das Auftreten von Überempfindlichkeitsreaktionen oder Allergien bzw. anaphylaktischen Reaktionen, zumal die dafür verantwortlichen basophilen Granulozyten und Mastzellen Oberflächenrezeptoren enthalten, an die der Fc-Teil von IgE bindet. Durch das Binden eines entsprechenden Antigens an den Fab-Anteil eines durch den Fc-Anteil an eine Mastzelle gebundenen IgE-Antikörpers werden durch diese Zelle Mediatoren freigesetzt, die die bekannten allergischen Reaktionen mit zum Teil schwerwiegenden Verlauf (z.B. Asthma, anaphylaktischer Schock) auslösen.

Von dem IgD ist bekannt, daß es an der Differenzierung der B-Lymphozyten beteiligt ist und bei Autoimmunprozessen eine Rolle spielen soll.

Es ist allgemein bekannt, daß die Wechselwirkung zwischen Antigen und Antikörper zu verschiedenen Phänomenen führt, von denen erfindungsgemäß insbesondere nur solche interessieren, bei denen das zur Sichtbarmachung der Reaktion dienende Antigen in partikulärer Form vorliegt. Als Ergebnis einer solchen Antigen-Antikörper Wechselwirkung kommt es zur Agglutinierung, und eine dafür notwendige Quervernetzung der antigenen Moleküle kommt nur zustande, wenn das Antigen multivalent und der Antikörper mindestens bivalent ist, gleichgültig ob letzterer intakt oder als Fragment, beispielsweise als F(ab')2-Fragment, vorliegt. Agglutination ist somit ein Phänomen, welches auf quervernetzten Antigen-Antikörper Komplexen beruht, deren Spezifität durch den engen Kontakt zwischen Epitop und spezifischen Antikörpern gleich dem Schlüssel-Schloß-Prinzip bedingt wird. Demgemäß führt die Reaktion eines Antikörpers mit einem multivalenten, unlöslichen Antigen zu einer Quervernetzung der Antigen-Partikel durch die Antikörper, welches zum mit dem bloßen Auge sichtbaren Phänomen der Agglutination führt. So kann beispielsweise eine Agglutinierung eines multivalenten unideterminanten Antigens (mit einem bestimmten Epitop) mit einem für dieses Epitop spezischen Antikörper erfolgen, oder wenn ein multideterminantes Antigen (mit verschiedenen antigenen Determinanten bzw. Epitope ) mit entsprechenden, spezifischen Antikörpern reagiert. Diese Reaktion ist abhängig von der Verdünnung der zu testenden Probe (z.B. Serum) im Verhältnis des zugegebenen Antikörpers (Antiserums), sodaß nur in einem speziellen Verdünnungsbereich der Probe eine Agglutination auftritt - ähnlich wie sie in der bekannten HEIDELBERGER-Kurve ihren Ausdruck findet. Die stärkste Verdünnung der zu testenden Probe, die noch eine Agglutination aufweist und nach der keine solche Reaktion mehr auftritt, wird als Titer bezeichnet und kann als Vergleich der relativen Konzentration agglutinierenden Antikörper in Proben (beispielsweise Körperflüssigkeiten, Seren) herangezogen werden.

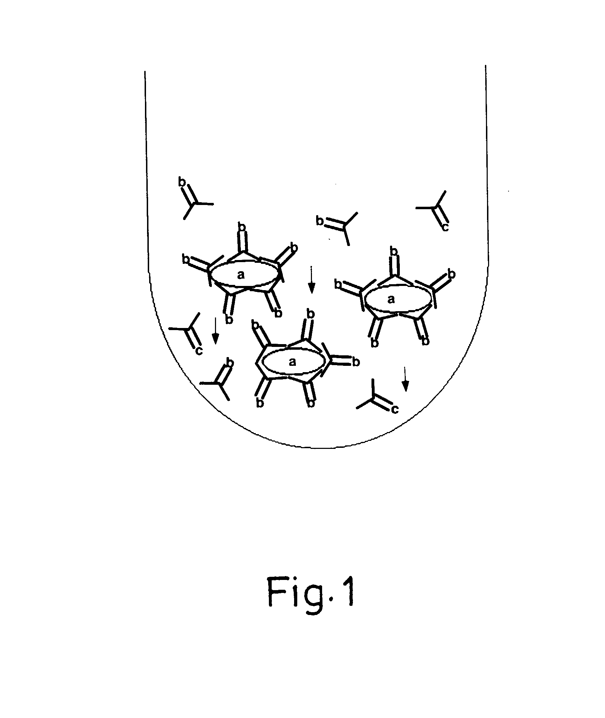

Das Zustandekommen der Agglutinationsreaktion ist auch insofern stark von der Antikörperkonzentration abhängig als nämlich die Agglutination ausbleibt, wenn eine bestimmte Antikörperkonzentration überschritten wird; selbst dann, wenn relativ große Mengen des entsprechenden Testantigens im Testansatz verwendet werden. Dieses Ausbleiben einer Agglutination wird als Prozone bezeichnet, worin die Antikörper in einem solchen Überschuß vorliegen, daß das Verhältnis Antigen zu Antikörper im Ungleichgewicht ist. Vermutlich liegt diesem Phänomen ein weitgehendes Abdecken der verfügbaren antigenen Determinanten durch die Antikörper zugrunde (Fig. 1). In jedem Fall kann dieses Prozonen-Phänomen zu falschen Ergebnissen führen, sodaß das Ausbleiben einer Agglutination nicht zwingend auf einen Mangel an entsprechenden Antikörpern deuten muß, sondern das Vorhandensein einer Prozone bedeuten kann. In Figur 1 wird das Prozonen-Phänomen, wie es bei der herkömmlichen, konventionellen direkten Agglutination auftritt, schematisch verdeutlicht.

Die Agglutination partikulärer Antigene durch Antikörper ist als bekannte Methode die einfachste und schnellste Weise, um Antikörperkonzentrationen in beispielsweise einem Serum zu bestimmen (z.B. quantitative Agglutinationsreaktion nach GRUBER und WIDAL). Hierbei liegen die Antigene entweder als natürliche Bestandteile auf der Oberfläche von z.B. Mikroorganismen (Bakterien) oder parasitären Einzellern oder die Antigene werden auf die Oberfläche von natürlichen Partikeln bzw. Zellen (beispielsweise Erythrozyten, Mikroorganismen) oder synthetischen inerten Partikeln oder organischen und anorganischen Trägern, wie z.B. Latex bzw. Polystyrol-Latex, Bentonit, Kohlenhydrate wie z.B. Zellulose oder Sepharose, Glasperlen, Styrole und Derivate davon, Liposomen, Metalloxidpartikel, Holzkohlepartikel oder Gelatine gebunden. Andere Techniken bedienen sich der Quervernetzung bzw. Polymerisierung löslicher Antigene, wodurch diese ebenso unlöslich gemacht werden können. Beispielsweise durch Verwendung von Ethyl-Chlorkohlensäureester (AVRAMEAS, S. & TERNYNCK, T., J. Biol. Chem. 242, 1651 - 1659, 1967), Glutaraldehyd (AVRAMEAS, S. & TERNYNCK, T., Immunochemistry 6, 53, 1969) oder von Ethylenmaleinsäureanhydrid (CENTENO, E. R. & SEHON, A. H., Immunochemistry 8, 887, 1971). Auf diese Weise kann die Agglutinierungsreaktion nicht nur für partikuläre Antigene Anwendung finden, sondern auch für a priori lösliche Antigene. Die letztgenannte Methode, das Kuppeln löslicher Antigene an entsprechende Partikel wird allgemein als passive Agglutination bzw. als passive Hämagglutination, wenn Erythrozyten Träger der Antigene sind, bezeichnet - im Gegensatz zur direkten Agglutination mit a priori partikulären Antigenen.

Die Agglutination wird seit langem als eine der wichtigsten serologischimmunologischen Methoden, insbesondere in der klinischen Diagnostik, angewendet und hat wegen ihrer relativ einfachen Handhabung eine breite praktische Bedeutung beim Nachweis von Antikörpern oder auch von Antigenen. So gehört zu den klassischen Anwendungen beispielsweise der Nachweis von Serumantikörpern gegen verschiedene Mikroorganismen wie z.B. bei Salmonellosen, Brucellosen, Listerosen, Rickettsiosen und anderen bekannten Infektionskrankheiten. Mit Antigen beladene Erythrozyten oder inerte Partikel anderer Natur dienen in vielbekannten diagnostischen Testsystemen dem Nachweis von Antikörpern. Die durch Antikörper ausgelöste Agglutination von beispielsweise mit Hormon beladenen Partikeln wird in Gegenwart von Hormon dosisabhängig gehemmt (Agglutinationshemmung); Hormone können somit nachgewiesen werden, beispielsweise das Choriongonadotropin im Harn zum immunologischen Schwangerschaftsnachweis.

Die Agglutinationstechnik wird im allgemeinen als Titrationsverfahren durchgeführt, wobei vorzugsweise eine mit Vertiefungen vorgesehene, transparente feste Phase, wie z.B. Röhrchen oder Titrationsplatten, Verwendung findet. Zu den bisher bekannten Verfahren zur Antikörper- oder Antigenbestimmung über Agglutinationsreaktionen zählen die bekannten Direktagglutinationen (z.B. nach WIDAL), bei denen die Antigenkomponente korpuskulär vorliegt - oder die passiven Agglutinationen, mit an inerten Partikeln gebundenen oder adsorbierten Antigenen.

Gemäß der DE-PS 2455369 werden Objektträger mit mindestens einer Vertiefung offenbart, auf dem ein immunologisch reaktives Material in lyophilisierter Form aufgebracht wird, wobei das immunologisch reaktive Material Antikörper oder insbesondere alle möglichen Antigene umfassen kann. Als ganz besonders geeignete immunologisch reaktive Materialien werden darin Toxoplasma gondii, Trypanosoma cruzi und Entamoeba histolytica genannt. Derartige, mit Antigen beschichtete Träger sollen für alle diagnostische Methoden, bei denen ein immunologisch aktives Material auf einen Träger in lyophilisierter Form aufgetragen wird, Verwendung finden. Demgemäß wird auf den Träger eine Serumprobe aufgetragen, diese nach Inkubation durch einen ersten Waschschritt entfernt und anschließend ein Antihumanglobulin, vorzugsweise mit einem Immunofluoreszenzfarbstoff markiert, hinzugegeben, worauf ein zweiter Waschschritt folgt.

In der DE-OS 3524451 werden Formkörper aus transparentem und wasserundurchlässigem Material mit einem Überzug aus Polymer beschrieben, worauf eine oder mehrere Komponenten von bioaffinen Bindungspartnern gebunden werden können. Ein derartiger Formkörper kann als Röhrchen oder flächig ausgestaltet sein und in letztgenannter Ausgestaltung mindestens eine Vertiefung oder Mulde aufweisen. Als Komponenten von bioaffinen Bindungspartnern werden unter anderem Partikel viralen oder zellulären Ursprungs, Proteine oder Peptide genannt.

Die Europäische Patentschrift EP-B1 0040572 beschreibt ein Verfahren zur Herstellung von Toxoplasmapräparationen, um nicht spezifische Agglutinationen zu verhindern.

In der EP-B1 0072319 wird ein Reagenz zur Diagnose von Toxoplasmose über direkte Agglutination beschrieben, wonach die Toxoplasmen mit einem Farbstoff, insbesondere mit einem Fluoreszenzfarbstoff, angefärbt werden, um das Sichtbarmachen einer Agglutinationsreaktion zu erleichtern.

Die EP-B1 008473 offenbart ein Verfahren zum Nachweis eines antigenspezifischen Immunglobulins IgX, wonach das Testmedium mit einem Anti-IgX gemischt und anschließend mit einem Antigen inkubiert wird, und nachfolgend mit einem markierten antigenbindenden Fragment eines spezifischen Antikörpers. Es wird beschrieben, daß die anti-IgX Komponente auf der Oberfläche eines entsprechenden Trägers (Röhrchen, Mikrotiterplatte) gekoppelt sein kann.

Die WO 91/04492 beschreibt einen Agglutinationstest, wonach die zu testende Probe in Portionen geteilt wird, welche getrennt mit entsprechenden Agglutinationsreagenzien reagieren, mit anschließender Wiedervereinigung der getrennten Proben für die eigentliche Agglutination.

In konventionellen, indirekten Agglutination-lmmunotest Systemen werden agglutinierbare Partikel, beispielsweise Erythrozyten, mit einem Antigen beladen ("gecoatet") und mit sowohl einem löslichen Antikörper als auch mit der zu testenden Probe zusammengebracht.

Hinsichtlich der Agglutinationsverfahren zur speziellen immundiagnostischen Erfassung von Protozoen, insbesondere Toxoplasmen, wurden neben der direkten Agglutination, beispielsweise nach DESMONTS, G. & REMINGTON, J. S., J. Clin. Microbiol. 11, 562 - 568, 1980, ein Immunsorbent Agglutination Assay (ISAGA) entwickelt (DESMONTS, G. et al., J. Clin. Microbiol. 14, 486 - 491, 1981), wonach ein Antiserum oder Antikörper gegen Immunglobuline der IgM Klasse an der Oberfläche der Vertiefungen von Mikrotiterplatten immobilisiert werden und nach Waschen das Testserum hinzugegeben wird. Nach einem erneuten Waschschritt werden anschließend die Toxoplasmen dazugegeben, worauf im positiven Fall bei Anwesenheit spezifischer IgM-Antikörper im Testserum eine Adsorption der Toxoplasmen an die feste Phase erfolgt, die als positives Ergebnis gewertet wird (auch: SAATHOFF, M., mta praxis 31, 721- 732, 1985).

Von GUPTA, S.K. et al., J. Immunol. Methods 80, 177 - 187, 1985, wird ein kompetitiver Erythro-lmmunoassay vorgeschlagen, wonach ein Antigen in einer Testprobe mit den Antigen-gekoppelten Erythrozyten um die Bindung an auf Vertiefungen von Mikrotiterplatten immobilisierten (gecoateten) monoklonalen oder chimaeren Antikörpern konkurrieren.

Im allgemeinen sind die beschriebenen herkömmlichen Agglutinationstests schneller durchzuführen, aber weniger sensitiv und in ihren Ergebnissen weniger eindeutig als die aufwendigeren Radioimmunoassays (RIA) oder die Enzymimmunoassays (EIA, ELISA), die auch als Festphasenimmunoassays bekannt sind, wobei in diesen Fällen Protein- bzw. Antigenmoleküle, gegen die entsprechende Antikörper reagieren, an Oberflächen adsorbiert worden sind.

Die japanische Patentanmeldung JP-A-01069954 (Zusammenfassung) offenbart ein Verfahren für einen Immunoassay, wonach die zu untersuchende Probe in ein Gefäß gegeben wird, in welchem Antikörper oder Antigene an dessen inneren Wand immobilisiert sind. Dazu werden mit entsprechend korrespondierenden Antikörpern oder korrespondierenden Antigenen beladene inerte Partikel hinzugefügt, wobei die zu untersuchende Probe nicht gewaschen wird. Das in der Probe befindliche Antigen oder der darin befindliche Antikörper reagiert mit dem an dem Gefäß fixierten Antikörper oder Antigen. Aus dieser Druckschrift sind jedoch keine weiteren Angaben, beispielsweise hinsichtlich des Waschens der Gefäße nach Hinzugabe der Proben und Testantigene, zu entnehmen.

Die bisher beschriebenen Agglutinationstests haben jedoch die entscheidenden Nachteile, daß sie entweder wenig sensitiv und/oder deren Ergebnisse durch das Auftreten einer Prozone unsicher - d.h. falsch-negativ bzw. fraglich-positiv - und/oder aufwendig durchzuführen sind, wobei ein relativ hoher Verbrauch an immunreaktiven bzw. immunologisch aktiven Material (Antigene, Antikörper) ein weiterer Nachteil ist. Insbesondere können diese Verfahren häufig durch unspezifische Antikörper (wie z.B. Antikörper der IgM-Klasse bzw. "natürliche Antikörper") oder solchen, welche gegen die korpuskulären Träger der Antigenpräparationen, z.B. gegen die Testerythrozyten im indirekten Hämagglutinationstest, gerichtet sind (z.B. heterophile Antikörper), beeinträchtigt sein.

Die Aufgabe der vorliegenden Erfindung war es, die oben dargelegten Nachteile des Standes der Technik zu beseitigen und ein bestehendes Bedürfnis nach einem schnellen, hochempfindlichen, sehr spezifischen, leicht durchzuführenden und aussagesicheren immunologischen Test zu befriedigen, welcher auf dem Prinzip einer Agglutinationsreaktion zwischen Antigenen und Antikörpern beruht.

Gelöst wurde die Aufgabe durch ein immunologisches Verfahren gemäß Anspruch 1, welches dadurch gekennzeichnet ist, daß ein nicht an eine feste Phase fixiertes immunologisch aktives Material in Kontakt gebracht wird mit auf einer festen Phase fixierten, d.h. immobilisierten (gecoateten) Antigenen und an der selben festen Phase fixierten, d.h. immobilisierten Antikörpern und daß die Agglutinationsreaktion ohne einen vorherigen Waschschritt erfolgt. Als immunologisch aktives Material kann auch eine geeignete, unlösliche oder unlöslich gemachte Antikörperpräparation vorliegen. Die Antigen- und/oder Antikörperpräparationen sind vorzugsweise unlöslich, insbesondere liegen sie in korpuskulärer Form vor.

Überraschenderweise wurde festgestellt, daß mit einem solchen immunologischen Testverfahren, welches ohne irgendeinen vorhergehenden Waschschritt durchgeführt wird, eine hinsichtlich der Agglutinationsreaktion erhebliche Sensitivitätssteigerung bei vereinfachter Durchführbarkeit, geringerem Antigenverbrauch und unter Vermeidung einer Prozone erzielt wird. Das Weglassen eines Waschschritts beim vorliegenden Verfahren ist, neben dem Immobilisieren ("Coaten") der Antigene und Antikörper auf einer festen Phase, ein erfindungswesentliches technisches Merkmal der vorliegenden Erfindung.

Gemäß dem erfindungsgemäßen Verfahren, welches auch unter dem Sammelbegriff "direkte Immunfixation-Agglutination" bezeichnet werden kann, werden in einem ersten Beschichtungsschritt an die Oberfläche einer festen Phase ein erstes immunologisch aktives Material immobilisiert bzw. fixiert (gecoatet), vorzugsweise Antikörper gegen Immunglobuline der zu untersuchenden Spezies, beispielsweise Antihuman-IgX, worin X die Bedeutung G, M, A, D, und/oder E hat. In einem zweiten Beschichtungsschritt wird ein zweites immunologisch aktives Material, welches vorzugsweise eine geeignete Antigenpräparation ist, wenn das im ersten Beschichtungsschritt immobilisierte immunologisch aktive Material ein Antikörper ist, an die noch freien Plätze der selben festen Phase gecoatet (immobilisiert, fixiert) werden. Es wird vorzugsweise mit der Präparation des geringeren Proteingehalts, in der Regel mit der Antikörperpräparation, begonnen. Anschließend wird die noch verbliebene freie Kapazität der Oberfläche derselben festen Phase durch ein geeignetes an sich bekanntes Blockierungsreagenz, vorzugsweise ein hinsichtlich der Herkunft des Antigens nicht verwandtes Fremdprotein, beispielsweise ein Serumalbumin (bovines Serumalbumin bei z.B. humanem Testantigen) oder Gelatine, in an sich bekannter Weise blockiert.

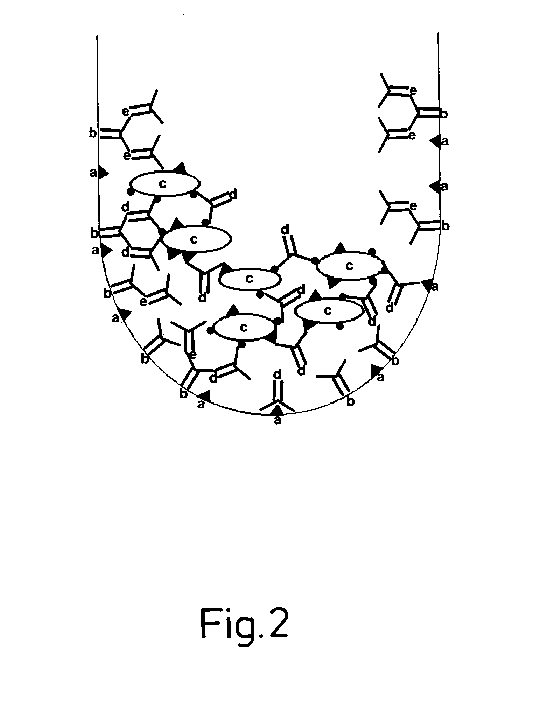

Die folgende Beschreibung des Testablaufs gemäß vorliegender Erfindung bezieht sich auf den Fall, daß die feste Phase des Testsystems mit verschiedenen immunologisch aktiven Materialien präpariert (gecoatet) wurde. Im Testansatz wird eine Untersuchungsprobe, welche biologischer oder nicht-biologischer Art sein kann, vorzugsweise eine Körperflüssigkeit eines Säugetieres bzw. Menschen, in welcher bei Bedarf vorher durch geeignete und bekannte Maßnahmen Störfaktoren bzw. unerwünschte Immunglobulinklassen (beispielsweise IgM) in an sich bekannter Weise eliminiert worden waren (beispielsweise durch 2-Mercaptoethanol, Dithiotreitol), mit einer Suspension der an inerten Partikeln gebundenen bzw. adsorbierten, korpuskulären Antigenpräparation gemischt. Bei Anwesenheit antigenspezifischer Antikörper in der Untersuchungsprobe kommt es zu einer Reaktion zwischen den an die feste Phase fixierten, beispielsweise human IgX-spezifischen, Antikörpern (sogenannte capture-Antikörper), den in Lösung befindlichen nachzuweisenden Antikörper (beispielsweise humanes IgX) und dem in der Lösung des Testansatzes befindlichen korpuskulären Testantigen. Außerdem bilden sich Immunkomplexe zwischen den an der Oberfläche der festen Phase fixierten Antigenen, den spezifischen Antikörpern der Untersuchungsprobe und dem korpuskulären Testantigen aus und es bilden sich Immunkomplexe zwischen verschiedenen Elementen des nicht-fixierten korpuskulären Antigens und den spezifischen Antikörpern der Untersuchungsprobe. Es kommt insgesamt zu einer mehrdimensionalen Immunreaktion mit dem entscheidenden Vorteil, daß durch die Bindung spezifischer Antikörper der Untersuchungsprobe an die feste Phase ein Antikörperüberschuß in der flüssigen Phase vermieden wird, ohne daß dadurch eine Empfindlichkeitseinbuße resultiert. Die Reaktion des korpuskulären Antigens mit den unterschiedlich an die feste Phase fixierten spezifischen Antikörpern führt vielmehr im Vergleich zu einem mit dem identischen korpuskulären Antigen durchgeführten Agglutinationstest zu einer erheblichen und überraschenden Sensitivitätssteigerung ohne daß sich die Gefahr einer Prozone ergibt. In Figur 2 ist dieser Sachverhalt schematisch verdeutlicht.

Voraussetzung für diese Testcharakteristik ist eine korrekte Abstimmung der Testkomponenten mit dem Effekt, daß auch niedrige Serumverdünnungen in diesem Testverfahren untersucht werden können und daher eine Vorverdünnung außerhalb des Testsystems nicht notwendig ist. Eine korrekte Abstimmung der Testkomponente kann beispielsweise dadurch erfolgen, daß durch Kreuztitrationsvorversuche mittels bekannter Standard-Testproben und in an sich bekannter Weise, beispielsweise mit humanem Standardserum, die optimalen Konzentrationen der immunologisch aktiven Materialien für die Beschichtung der festen Phase ermittelt werden.

Das erfindungsgemäße Verfahren besitzt gegenüber den bekannten immunologischen Tests, insbesondere den Agglutinationstests, zusammmengefaßt die Vorteile
. kein Auftreten einer Prozone, auch nicht bei niedrig verdünnter Untersuchungsprobe (z.B. Serum)
. hohe Sensitivität, ohne Einbuße der Spezifität
. einfache Durchführbarkeit (z.B. keine Vorverdünnung der Untersuchungsprobe)
. geringerer Antigenverbrauch
. keine zusätzlichen Waschschritte
. um einige Zehnerpotenzen weiterer Meßbereich gegenüber z.B. Immunfluoreszenztest oder ELISA.

Das erfindungsgemäße Verfahren ist vorteilhafterweise zum Zwecke einer Bestimmung aller möglichen Antikörper bei bekannten Antigenen und vice versa ebenso zur Bestimmung von Antigenen bei bekannten Antikörpern, beispielsweise in Form des Neutralisations- bzw. Agglutinations-Hemmtests, geeignet. Dieser Sachverhalt ist in Figur 2 schematisch näher dargestellt. Die Anwendung des erfindungsgemäßen Verfahrens beschränkt sich daher nicht nur auf das Gebiet der Infektionsdiagnostik als solche, sondern umfaßt allgemein das Suchen, Erkennen und Bestimmen jedes beliebigen Antigens (Antigen-Screening und Antigen-Diagnostik) zum Erfassen bzw. Diagnostizieren eines physiologischen Zustandes biologischer Systeme, beispielsweise eines Säugetieres bzw. Menschen (z.B. Schwangerschaftsnachweis), eines nicht regelhaften Zustandes eines biologischen Systems, beispielsweise eines pathologischen körperlichen Zustandes bzw. einer Erkrankung eines Säugetieres bzw. Menschen (z.B. Tumordiagnostik, Infektionsdiagnostik) oder eines durch Einnahme von pharmakologisch wirksamen Wirkstoffen verursachten Zustandes (z.B. Medikamentenmißbrauch, Nachweis von Medikamenten- oder Drogenmetaboliten oder von Wirkstoff- oder Metabolitenakkumulationen, Halbwertszeitbestimmung von pharmakologisch wirksamen Stoffen, Bioverfügbarkeit und Clearance derselben, Medikamenten- oder Drogenabhängigkeit) in einem biologischen System, einem Säugetierorganismus bzw. in einem Menschen, soweit diese Zustände durch das Auftreten von für sie spezifischen Antigenen und/oder Antikörpern begleitet und gekennzeichnet sind. Darüber hinaus kann eine zu untersuchende Probe ein nicht-biologisches flüssiges Medium sein, soweit dieses agglutinierbare Antigene und/oder Antikörper enthält.

Gegenstand der vorliegenden Erfindung ist nicht nur das Verfahren gemäß Anspruch 1 und seiner näheren Ausgestaltungen gemäß der weiteren abhängigen Ansprüche dieser Kategorie, sondern auch die Verwendung der für das erfindungsgemäße Verfahren geeigneten Formkörper und Vorrichtungen einschließlich eines Kits (Testverpackung, Testystem) und/oder ein kit-of-parts und diese Formkörper und Vorrichtungen, Kits und kit-of-parts als solche.

Der für die vorliegende Erfindung wesentliche Begriff "feste Phase", an die erfindungsgemäß das immunologisch aktive Material immobilisiert bzw. fixiert (gecoatet) wird (beispielsweise durch kovalente Bindung oder durch Adsorption), umfaßt jedes wasserunlösliche Material, von dem der Fachmann weiß, daß es Proteine und Peptide auf seiner Oberfläche binden bzw. adsorbieren kann, vorzugsweise solches, welches aus Licht-transparenten Materialien (beispielsweise Kunststoffe wie Polyvinyle, Polystyrole, Acrylate, Glas oder Mineralien) besteht. Derartige Materialien können von unterschiedlicher Form und Größe sein, je nachdem, wie sie für den Fachmann individuell jeweils vorteilhaft gemäß der vorliegenden Erfindung angewandt werden. Vorzugsweise besteht das Material der festen Phase entweder aus an einem Ende dauerhaft verschlossenen Hohlkörper (beispielsweise Röhrchen, Tuben, kolbenförmige Behälter) oder aus mit mindestens einer Vertiefung oder Mulde, die gerundet oder eckig ausgestaltet sein können und in denen sich das immunologisch aktive Material in immobilisierter Form adhäriert oder in Suspension bzw. Lösung befindet, versehenen Formkörper, der vorzugsweise flächig ausgestaltet sein kann (beispielsweise Platten, Scheiben, Tafeln, Streifen, Objektträger, Mikrotiterplatten).

Der erfindungsgemäß verwendete Begriff "immunologisch aktives Material" umfaßt sowohl Immunglobuline bzw. Antikörper und Derivate davon als auch Antigene bzw. Antigenpräparationen, soweit diese zur Bindung der interessierenden Antikörper bzw. zur Agglutinationsreaktion befähigt sind.

Im Sinne der vorliegenden Erfindung sind unter dem Begriff "Immunglobuline" oder synonym dafür "Antikörper", mono- und zumindest bivalente bzw. polyvalente, poly- und monoklonale Antikörper zu verstehen, insbesondere der Isotypen IgG, IgM, IgA, IgE, und IgD und deren Unterklassen; aber auch solche, die Fragmente davon darstellen und Derivate davon, einschließlich der F(ab')2, Fab', Fab und Fc Fragmente, aber auch chimäre Antikörper oder Hybridantikörper mit mindestens zwei Antigen- bzw. Epitopbindungsstellen, oder bispezifische rekombinante Antikörper (beispielsweise Quadrome, Triome), Interspecies-Hybridantikörper, anti-idiotypische Antikörper und solche davon, die chemisch modifiziert wurden und als Derivate dieser Antikörper zu verstehen sind und die entweder über die bekannten konventionellen Verfahren der Antikörpergewinnung oder über DNA-Rekombination, via Hybridomatechnik oder Antikörper-Engineering oder synthetisch oder semisynthetisch nach an sich bekannten Verfahren herstellbar sind und Neutralisierung-, Agglutinations- oder Bindungseigenschaften hinsichtlich des im weiteren noch näher erläuterten und definierten Begriffs "Antigen" aufweisen. Aus der vielfältigen Literatur sei nur beispielhaft hingewiesen auf Arbeiten von KÖHLER, G. & MILSTEIN, C., Nature 256, 495 - 497, 1975; BIOCCA, S. et al., EMBO J. 9, 101 - 108, 1990; BIRD, R.E. et al., Science 242, 423 - 426, 1988; BOSS, M.A. et al., Nucl. Acids Res. 12, 3791 - 3806, 1984; BOULIANNE, G.L. et al., Nature 312, 643 - 646, 1984; BUKOVSKY, J. & KENNETT, R.H., Hybridoma 6, 219 - 228, 1987; DIANO, M. et al., Anal. Biochem. 166, 223 - 229, 1987; HUSTON, J.S. et al., Proc. Natl. Acad. Sci. USA 85, 5879 - 5883, 1988; JONES, P.T. et al., Nature 321, 522 - 525, 1986; LANGONE, J.J. & VUNAKIS, H.V. (Hrsg.), Methods Enzymol. 121, Academic Press. London, 1987; MORRISON, S. et al., Proc. Natl. Acad. Sci. USA 81, 6851 - 6855, 1984; 01, V.T. & MORRISON, S.L., BioTechniques 4, 214 - 221, 1986; RIECHMANN, L. et al., Nature 332, 323 - 327, 1988; TRAMONTANO, A. et al., Proc. Natl. Acad. Sci. USA 83, 6736 - 6740, 1986; WOOD, C.R. et al., Nature 314, 446 - 449, 1985.

Hinsichtlich der Herstellung von polyklonalen Antikörpern stehen ebenfalls eine Anzahl bekannter Verfahren zur Verfügung. Es können z.B. für diesen Zweck in an sich bekannter Weise verschiedene Tiere durch Injektion mit einem geeigneten bzw. gewünschten Antigen, welches natürlichen Ursprungs, über DNA-Rekombination oder synthetisch bzw. semisynthetisch hergestellt sein kann, oder Fragmente davon, immunisiert werden und aus den danach gewonnenen Seren die gewünschten polyklonalen Antikörper nach an sich bekannten Methoden gewonnen und gereinigt werden. Als Alternative können auch intakte Zellen benutzt werden. Verschiedene Adjuvantien zur Erhöhung der Immunantwort auf die Antigen-Gabe können, abhängig von dem für die Immunisierung ausgewählten Tier, ebenfalls verwendet werden - beispielsweise Freund's Adjuvant, Mineralgele wie z.B. Aluminiumhydroxid,, oberflächenaktive Substanzen wie z.B. Polyanionen, Peptide, Ölemulsionen, Hemocyanine, Dinitrophenol oder Lysolecthin.

Die für die erfindungsgemäße Verwendung zu benutzenden monoklonalen Antikörper können durch jede beliebige, bekannte Technik erhalten werden, die für die Herstellung von Antikörpern über Kultivierung von Zellinien zur Verfügung stehen. Zu derartigen bekannten Techniken zählen beispielsweise die von KÖHLER, G & MILSTEIN C., 1975, Ioc. cit., oder TAGGART & SAMLOFF, Science 219, 1228 - 1230, 1983, beschriebenen Verfahren mit Hybridomazellen oder solche mit humanen B Zell Hybridomen (KOZBOR et al., Immunology Today 4, 72 - 79, 1983). Chimäre Antikörper können beispielsweise aus einer Maus-Antigen-Bindungsdomäne und humanen konstanten Regionen zusammengesetzt sein (MORRISON, et al., Proc. Natl. Acad. Sci.USA 81, 6851 - 6855, 1984; TAKEDA, S. et al., Nature 314, 452 - 454, 1985). Chimäre Antikörper können beispielsweise gemäß der europäischen Patentanmeldung EP-A 0481 502 hergestellt werden.

Die gewonnenen Antikörper können nach bekannten Methoden gereinigt werden, beispielsweise über Gelfiltration, Ionenaustauschchromatographie, Immunoabsorptions- oder Immunoaffinitätschromatographie, über HPLC (High Performance Liquid Chromatography) oder Kombinationen davon. Antikörper Fragmente, welche den Idiotyp des Moleküls enthalten, können gleichermaßen nach bekannten Verfahren hergestellt werden. Beispielsweise können F(ab')2 Fragmente und Fc' Fragmente durch Pepsin Verdauung des vollständigen poly-oder monoklonalen Antikörpers erhalten werden. Fab' Fragmente können erhalten werden, indem beispielsweise die Disulfidbrücken des betreffenden F(ab')2 Fragments reduziert werden und Fab als auch Fc Fragmente können geschaffen werden beispielsweise durch Behandlung der Antikörpermoleküle mit Papain und einem Reduktionsmittel. Derartige Verfahren sind dem Fachmann bekannt und gehören zum Stand der Technik.

Zur Identifizierung und Selektion von Antikörpern, Fragmenten oder Derivaten davon, die mit mindestens einem Epitop des entsprechenden Antigens reagieren, kann jedes bekannte Verfahren verwendet werden. Beispielsweise dadurch, daß diese nach entsprechender Markierung detektierbar sind, wenn sie an isoliertes oder gereinigtes Antigen gebunden haben oder über Immunpräzipitation des beispielsweise über Gelfiltration, Chromatographie oder Polyacrylamidgele gereinigten Antigens, oder dadurch, daß Antikörper gegen ein entsprechendes Antigen mit anderen Antikörpern um das Binden an dasselbe Antigen konkurrieren.

Zur weiteren Konkretisierung der oben schon erläuterten, breiten Verwendbarkeit des erfindungsgemäßen Verfahrens zur Diagnostik verschiedener körperlicher Zustände und Erkrankungen durch Verwendung biologischer Flüssigkeiten und zur Bestimmung von immunologisch aktiven Material in nicht-biologischen Flüssigkeiten soll der Begriff "Antigen" bzw. "Antigenpräparation" weiter näher definiert werden.
Erfindungsgemäß umfaßt der Begriff "Antigen" in biologischen Flüssigkeiten bzw. Untersuchungsproben solche, die in einem Säugetierorganismus bzw. Menschen extrazellulär, intrazellulär, transzellulär (Third space) oder interstitiell vorkommen, wie beispielsweise Blut, Serum, und Fraktionen davon, Exkremente (wie z.B. Urin, Faeces), Tränenflüssigkeit, Speichel, Amnionflüssigkeit, Gewebeflüssigkeit, Aszites, Samenflüssigkeit, Pleuraflüssigkeit, Cystenflüssigkeit, Cerebralflüssigkeit, Eiter, Sekrete des Magen-Darm-Trakts, Liquor, Augenkammerflüssigkeit, Ödemflüssigkeit, pathologisch auftretende Flüssigkeit bei z.B. Ileus oder Peritonitis, Sekrete, Zerebrospinalflüssigkeit, Plasma, Lymphe oder Flüssigkeiten, die aus Gewebeextrakten erhalten werden, Gewebe- und Zellkulturen.

"Nicht-biologische Flüssigkeiten" bzw. Untersuchungsproben können im Sinne der vorliegenden Erfindung Abwässer, Trinkwasser, stehende oder fließende Gewässer oder Grundwasser sein bzw. solche, die nicht an das Vorkommen in biologischen Systemen, wie z.B. Zellkulturen oder einem Säugetierorganismus gebunden sind und nicht-lösliches, partikuläres immunologisch aktives Material im obigen Sinne enthält oder dieses in löslicher Form vorliegt und bei Bedarf im nachhinein in bekannter Weise an Partikel gebunden bzw. adsorbiert oder nach den bekannten Methoden entsprechend chemisch behandelt und entsprechend modifiziert bzw. polymerisiert werden kann.

Als erfindungsgemäß in Betracht kommende Antigene und Antigenpräparationen können beispielhaft genannt werden, wobei diese Aufzählung keineswegs die weiteren bekannten Antigene ausschließen soll: Immunoglobuline bzw. Antikörper im Sinne der oben gegebenen Definition, Proteine, Polypeptide, Oligopeptide, Hormone (z.B. Steroidhormone wie z.B. Östrogene, Gestagene, Androgene, Glukokortikoide, Mineralokortikoide, Cholecalciferole, Proteohormone, biogene Amine, Oxytozin, Vasopressin (ADH), Insulin, Glukagon, Parathormon, Kalzitonin, Erythropoetin, Prostaglandine, Gonadotropine wie z.B. luteinisierendes Hormon (LH), Choriongonadotropin (HCG bzw. β-HCG), Follikel stimulierendes Hormon (FSH), Prolaktin und andere Plazentahormone, Serotonin, Histamin, Bradykinin, Kallikrein, gastrointestinale Hormone, Schilddrüsenhormone, Katecholamine, Azetylcholin), Enzyme, Tumormarker bzw. Tumorantigene (wie z.B. karzinoembryonales Antigen (CEA), alpha-1-Fetoprotein (AFP), CA 15-3, CA 19-9, CA-50, CA-125, Postataspezifische saure Phosphatase (PAP), neuronspezifische Enolase (NSE), Bence-Jones-Protein, alkalische Phosphatase (AP), Prostata-spezifisches Antigen (PSA), Hormone endokriner Tumore, LDH, Gamma-GT, Neopterin, Ferritin), Mikroorganismen wie Bakterien (beispielsweise Enterobacteriaceae, Corynebacterien, Spirochaeten, Pseudomonaden, Mycoplasmen, Coccaceae, Chlamydien, Bacillaceae), Pilze und Sporen davon (beispielsweise Candida, Cryptococcus, Blastomyces, Paracoccidoides), durch virale Infektionen von DNA- und RNA-Viren bedingte Antigene, beispielsweise virale Capsidproteine oder Hüllproteine (zum Beispiel Hepatitis Viren und verwandte Antigene wie z.B. HBe, HBc, HBs; HIV, Small pox, Pockenviren, Coxsackieviren, Echoviren, Myxoviren, Rhabdoviren, Togaviren, Reoviren, Tumorviren, Picornaviren, Herpesviren, Paramyxoviren, Rubellaviren, Mumpsviren, RS-Virus, Coronaviren), Protozoen (wie beispielsweise Leishmania, Trichomonas, Trypanosoma, Sarcocystidae mit Toxoplasma, insbesondere Toxoplasma gondii, Eimeriidae, Plasmodium, Cryptosporiidae), Drogen und Medikamente und Metabolite davon (beispielsweise Opiate und Opioide, Kokain, Diazepine, Barbiturate, Paracetamol, Theophylline), Gifte. Mitumfaßt sind auch über die bekannten Methoden der DNA-Rekombination hergestellten Antigene, beispielsweise wie sie für Toxoplasma gondii in der EP-A 0431541 beschrieben sind, und über die bekannten synthetischen und semisynthetischen Verfahren herstellbaren Antigene, aber auch diejenigen Antikörper, die gegen die oben genannten Antigene gebildet werden.
Ein für die vorliegende Erfindung ganz bevorzugtes Antigen sind Toxoplasmen, insbesondere Toxoplasma gondii, dessen diagnostische Erfassung auch heute noch ganz besondere Probleme aufweist, da die bisherige Serumdiagnostik auch in diesem Fall nicht immer verläßliche Daten liefert.

Als "inerte Partikel", an die die Antigene zu binden bzw. zu adsorbieren sind, um eine erfindungsgemäß zu verwendende partikuläre bzw. korpuskuläre Antigenpräparation zu erhalten, können alle dem Fachmann bekannten natürlichen, synthetischen, organischen oder anorganischen Partikel verwendet werden, die eine Agglutination mit entsprechenden Antikörpern bewirken, insbesondere Blutzellen (Erythrozyten, beispielsweise Toxocell IHA von Reditest,S.A.,Barcelona, oder Mast Serodia-Anti HBs FD 411 von Mast Diagnostica, Hamburg, oder gemäß BIRD, T. & STEPHENSON, J.H., J. clin. Path. 26, 623 - 627, 1973, oder Serodia-AFP von Fujizuki Pharmaceutical Co., Ltd., Japan), Latex bzw. Polystyrol-Latex (beispielsweise von Tokuyama Soda Co., Ltd., Japan, oder wie sie in der EP-A 0435851 beschrieben sind), Mikroorganismen (Bakterien und Pilze, beispielsweise Salmonellen, Candida albicans oder Yersinia gemäß Yersinia Agglutube der Firma Labor Diagnostika GmbH, Helden, Deutschland, oder Serratia gemäß SAUL, F. et al. Ärztl. Lab. 23, 407 - 411, 1977 (Blue-ASO-Test)), Bentonit (REISBERG, M.A. et al., J. Immunol. 105, 1151, 1970), Kohlenhydrate wie z.B. Zellulose (CAMPBELL, D.H. et al., Proc. Natl. Acad. Sci. USA 37, 575, 1951; WELIKY, N. & WEETALL, H.H., Immunochemistry 9, 967, 1972), Sepharose (WILCHEK, M. et al., Biochemistry 10, 2828, 1971), Glasperlen (WEETALL, H.H., J. Biochem. 117, 257, 1970), Styrole und Derivate davon (EP-A 0466170), Liposomen, Metalloxidpartikel, Holzkohlepartikel, Gelatine (beispielsweise Serodia-HIV von Fujirebio Inc., Tokyo, Japan). Es sei darauf hingewiesen, daß die Namen "Serodia" eingetragene Warenzeichen sind.

Erfindungsgemäß umfaßt werden aber auch a priori lösliche Antigene, die durch die bekannten Methoden unlöslich gemacht werden können, beispielsweise durch Polymerisation mit Ethylchlorkohlensäureester gemäß AVRAMEAS, S. & TERNYNCK, T., J. Biol. Chem. 242, 1651 - 1659, 1967, oder durch Verwendung von Glutaraldehyd nach AVRAMEAS, S. & TERNYNCK, T., Immunochemistry 6, 53, 1969, oder von Ethylenmaleinsäureanhydrid nach CENTENO, E.R. & SEHON, A.H., Immunochemistry 8, 887, 1971. Demzufolge kann die der vorliegenden Erfindung zugrundeliegende Agglutinationsreaktion nicht nur für a priori partikuläre Antigene oder für an inerte Partikel gebundene korpuskuläre Antigene Anwendung finden, sondern auch für a priori lösliche Antigene.

Ein Kit bzw. Testkit (Verpackung, Testsystem) kann vorzugsweise aus den folgenden Bestandteilen zusammengesetzt sein: erfindungsgemäß beschichtete, gebrauchsfertige feste Phase (Module bzw. Formkörper), eine bestimmte Menge eines Antigens und/oder eine bestimmte Menge eines Antikörpers bzw. Immunglobulins. Ein solcher Kit kann neben diesen Bestandteilen die zusätzlichen Komponenten enthalten: Verdünnungspuffer für das immunologisch aktive Material (Antikörper, Antigene), ein positives und/oder negatives Kontrollserum bzw. eine positive und/oder negative Antigenkontrolle, welches lyophilisiert sein kann und in diesem Fall für die Anwendung rekonstituiert werden muß (beispielsweise mit Aqua bidest.), ein Reduktionsreagenz (beispielsweise Dithiothreitol, DTT). Die genannten Einzelkomponenten können selbstverständlich auch in einer anderen Packungszusammensetzung oder -zusammenstellung oder auch einzeln verpackt vorliegen.

Legenden zu den Figuren:

Fig.1: Prozonen-Phänomen bei der konventionellen direkten Agglutination mit unbeschichteten Modulen. a = korpuskuläres (z.B. Toxoplasma)-Antigen (z.B. formalin-fixierte Parasiten), b = spezifische Patienten-IgX (z.B. IgG)-Antikörper, c = unspezifische Patienten-IgX (z.B. IgG)-Antikörper.

Fig. 2: Direkte Immunfixations-Agglutination unter Verwendung von Antigen-und Antikörper-beschichteten Modulen. a = fixiertes Antigen (z.B. Sarcosyl-Antigen), b = fixierter (capture) Antikörper (z.B. anti-human-lgG vom Kaninchen), c = korpuskuläres (z.B. Toxoplasma)-Antigen, beispielsweise Formalin-fixierte Parasiten, d = spezifische Patienten-IgX (z.B. IgG)-Antikörper, e = unspezifische Patienten-IgX (z.B. IgG)-Antikörper.

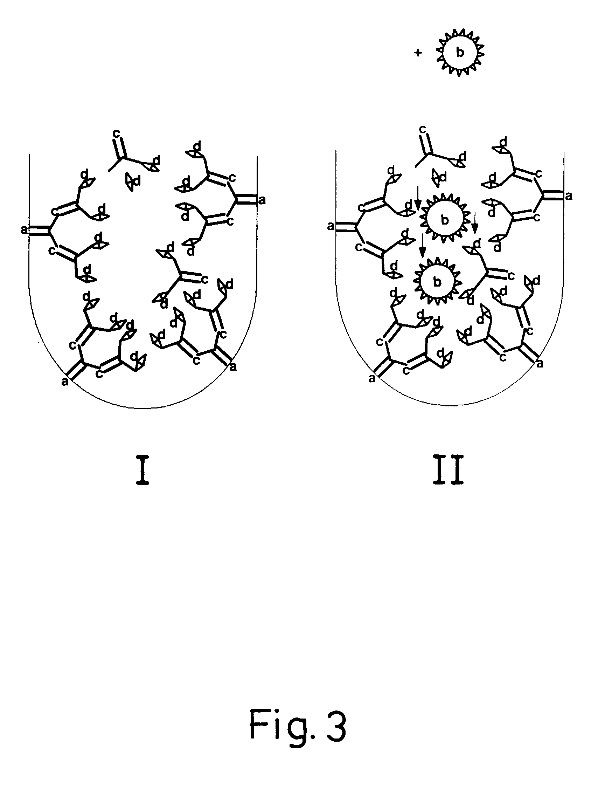

Fig.3: Immunfixations-Agglutinations-Hemmtest: das gesuchte Antigen in der Probe neutralisiert eine begrenzte Quantität eines Ag-spezifischen Antikörpers. = 1. Phase (Neutralisation), II = 2. Phase (Detektion), a = Capture-Antikörper mit Spezifität gegen Test-Antikörper, b = korpuskuläres Test-Antigen, c = Antigen-spezifische Test-Antikörper (bekannte Menge), d = gesuchtes Antigen in der Probe.

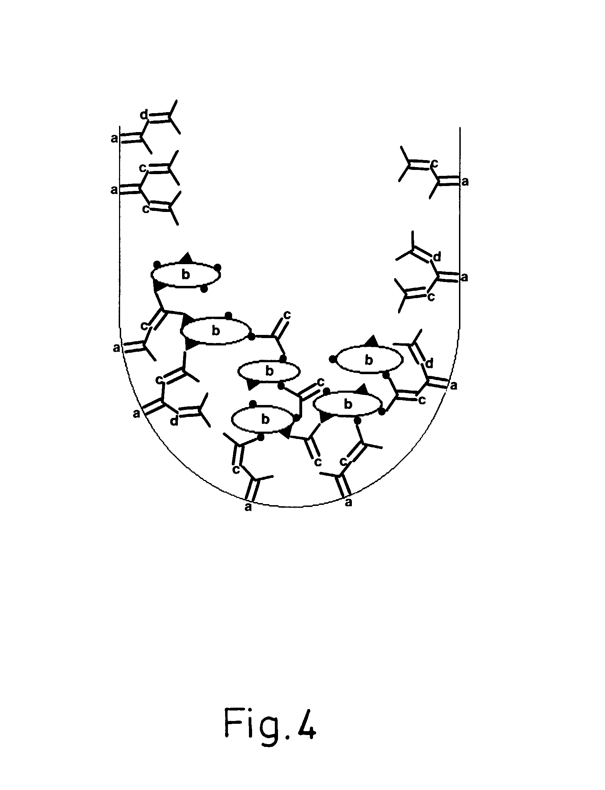

Fig.4: Direkte Immunfixations-Agglutination unter Verwendung von Antikörper-beschichteten Modulen. a = Capture-Antikörper (z.B. Anti-human-lgG vom Kaninchen), b = korpuskuläres (z.B. Toxoplasma)-Antigen (z.B. Formalin-fixierte Parasiten), c = spezifische Patienten-IgX (z.B. IgG)-Antikörper, d = unspezifische Patienten-IgX (z.B. IgG)-Antikörper.

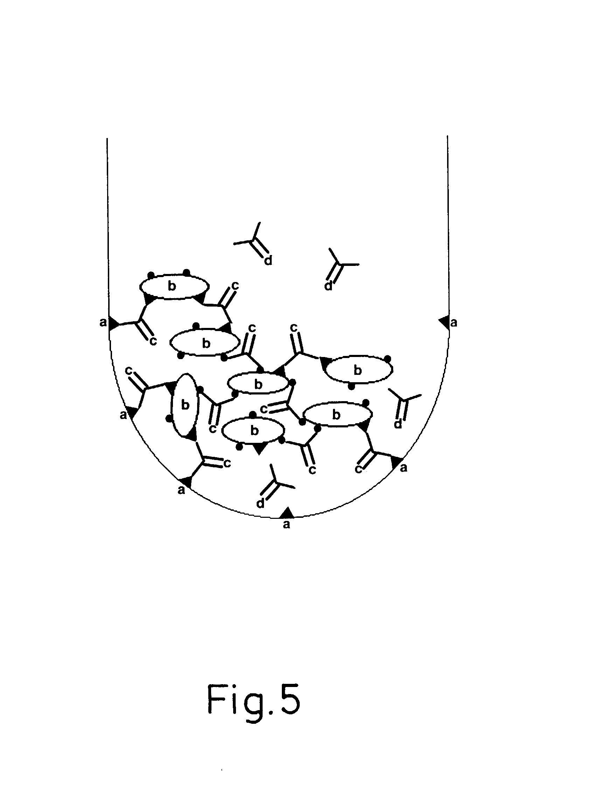

Fig.5: Direkte Immunfixations-Agglutination unter Verwendung von Antigen-beschichteten Modulen. a = fixiertes Antigen (z.B. Sarcosyl-Antigen), b = korpuskuläres (z.B. Toxoplasma)-Antigen (z.B. Formalin-fixierte Parasiten), c = spezifische Patienten-IgX (z.B. IgG)-Antikörper, d = unspezifische Patienten-IgX (z.B. IgG)-Antikörper.

Das folgende Beispiel soll die Erfindung in seiner konkretesten Form näher erläutern, ohne daß der Erfindungsgegenstand darauf zu beschränken wäre.

### Beispiel 1: Nachweis von Toxoplasma-spezifischen Antikörpern beim Menschen

### 1.1. Herstellung der Testmodule

U-Boden-Module mit hoher Proteinbindungskapazität (MaxiSorb, Fa. NUNC, Kamstrup, DK 4000 Roskilde, Dänemark) wurden sowohl mit einem Antiserum vom Kaninchen gegen humane IgG-Antikörper (anti-γ-Antiserum A090, Fa. Dakopatts, PF 70 04 07, D-2000 Hamburg 70) als auch mit einem Antigenextrakt aus *Toxoplasma gondii* beschichtet. Kreuztitrationen nach bekannten Maßnahmen unter Verwendung üblicher geeigneter Referenzseren erbrachten die optimalen Coatingverdünnungen, wobei folgendes Vorgehen gewählt wurde:
Das Anti-Human-lgG-Serum (Capture-Antiserum) als auch der Antigenextrakt wurden mit Coating Puffer verdünnt. Das Capture-Antiserum wurde in unterschiedliche geometrische Verdünnungsstufen übertragen (1:2000, 4000, 6000 und nur Coatingpuffer als Leerwert) zu je 100 µl in Kavitäten der U-Boden-Module. Gecoatet wurde über Nacht bei 4 - 8° C in einer feuchten Kammer. Als Coating-Puffer (CB) wurde ein alkalischer Carbonatpuffer mit pH 9,6 verwendet (14,7 g NaHCO₃, 8 g Na₂CO₃, 1 g Na-Azid in 5000 ml A. bidest, Einstellung des pH-Wertes durch 1 n NaOH - alle Substanzen wurden als Reinst-Chemikalien von der Fa. Merck, Darmstadt bezogen). Anschließend wurde 4 mal mit Phosphat-gepufferter Kochsalzlösung (PBS) gewaschen, ohne das die Module zwischenzeitlich austrockneten. Alle hier eingesetzten PBS-Puffer wurden nach Bedarf durch Mischen von 400 ml saurer PBS-Stammlösung I (59,4 g Na₂HPO₄, Fa. Merck, in 5000 ml A. demi.) mit 100 ml alkalischer PBS-Stammlösung II (22,7 g KH₂PO₄, Fa. Merck, in 2500 ml A. demi.) und 4500 ml A. demi. hergestellt, in welche 42,5 g NaCI (Fa. Merck) eingewogen wurden. Alle Kavitäten jeder Capture-Antiserum-Verdünnungen wurden im weiteren Verlauf im Sinne einer Kreuztitration mit 100 µl Toxoplasma-Antigen-Verdünnung (Antigenextrakt in CB) pro Vertiefung in der Verdünnungsstufen 1:800, 1:1600, 1:3200 und Leerwert (nur Coating Puffer) beschichtet. Der hier als Antigen verwendete Toxoplasma-Sarcosyl-Extrakt wurde bis zum Gebrauch bei -20° C gelagert und war vorher folgendermaßen hergestellt worden:
Weibliche NMRI-Mäuse (Fa. SAVO, D-7964 Kisslegg im Allgäu) im Alter von 8 - 16 Wochen wurden mit je 4,5 - 5,5 x 10⁶ Toxoplasmen (Maus-virulenter BK-Stamm) i.p. infiziert. Nach 2 Tagen wurden die Toxoplasmen aus dem Peritoneum durch Spülen mit 2 x 5 ml Hanks-Lösung (Fa. Seromed, Biochrom KG, D 1000 Berlin, Kat.Nr. L2013) pro Maus gewonnen. Die Exsudate wurden vor dem Mischen mikroskopisch kontrolliert. Das Verhältnis von Mauszellen/Toxoplasmen mußte <1:60 sein. Exsudate, die diesen Qualitätsanforderungen nicht entsprachen, wurden verworfen.
Die gepoolten verwendbaren Toxoplasmen wurden 3 mal mit Hanks-Lösung gewaschen (2000 U/min, 10 Minuten) und anschließend in kaltem Sarcosylpuffer (4° - 8° C) aufgenommen und 15 Minuten im Kühlschrank bei 4° - 8° C inkubiert. Der verwendete Sarcosylpuffer war vorher wie folgt hergestellt worden: Zu 1000 ml A. dest. wurden 1,21 g TRIS, O,29 g Titriplex und 2 % N-Laurosylsarcosin-Natriumsalz (67 ml einer 30 %igen Lösung) gegeben. Der pH wurde bei Bedarf mit 1 n HCI auf einen Wert von 8,0 eingestellt (alle genannten Reagenzien wurden von der Fa. Merck bezogen). 4 - 6 x 10⁸ Toxoplasmen wurden in 3 ml Sarcosyl-Puffer aufgenommen. Die Lösung wurde nach der Kühlschrank-Inkubation in einer Cryofuge bei 4° C und 20 000 U/min 15 Minuten abzentrifugiert, der Überstand abpipettiert und das Pellet verworfen (Überstand = "Toxoplasma-Sarcosyl-Antigen").
Die Testmodule wurden mit den oben beschriebenen unterschiedlichen Antigen-Verdünnungen für 3 Stunden bei Raumtemperatur in einer feuchten Kammer gecoatet, anschließend wieder 4 mal mit PBS gewaschen und (ohne daß die Module zwischenzeitlich austrockneten) mit 100 µl Blockpuffer (1 % (w/v) bovines Serumalbumin der Fa. Fluka, PF 1346, D-7910 Neu-Ulm und 0,1% (w/v) Na-Azid, Fa. Merck, in PBS) pro Kavität für zwei Stunden bei Raumtemparatur (RT) in einer feuchten Kammer abgeblockt. Nach dem Blockiervorgang wurden die Module 4 mal mit demineralisiertem Wasser gewaschen, auf einer sauberen Zellstoffunterlage ausgeklopft und bei 37° C unter der Verwendung von Trockenmittel (z.B. Silikalgel-Beutel) im Vakuum für 30 Minuten getrocknet und konnten bis zur weiteren Verwendung in einer Polyamid-Folie mit Trockenmittel eingeschweißt mindestens 9 Monate gelagert werden.
Durch Kreuztitration mit herkömmlichen Toxoplasma-Antikörper-positiven und - negativen Referenzseren sowie mittels eines IgG-Kalibrationsserums (auf das WHO-lgG-Referenzserum eingestellt) wurden optimale Coating-Konzentrationen festgelegt. Die Testdurchführung erfolgte entsprechend der weiter unten dargestellten Technik. Zusätzlich wurden durch ELISA unter Verwendung eines Anti-Kaninchen-lg-AP-Konjugates (D 306, Fa. Dakopatts) die Capture-Antiserum; Beschichtung und durch Verwendung üblicher IgG-positiven Maus-Seren und Anti-Maus-lgG-POD (Fa. Binding Site, Bezug über Fa. LD Labordiagnostika, Heiden) die Antigen-Beschichtung der Testmodule überprüft. Nach Bestimmung der optimalen Coating-Verdünnung erfolgte die Herstellung der einheitlich beschichteten Module analog der für die Kreuztitrationen beschriebenen
Technik, wobei allerdings aus Gründen der Praktikabilität der Coating-Vorgang für das Toxoplasma-Sarcosyl-Antigen über Nacht bei 4 - 8° C erfolgte.

### 1.2. Testantigen

Als Agglutinations-Antigen für den Test dienten formalinfixierte Toxoplasmen der Fa. LD Labordiagnostika Heiden (DA Direktagglutinationstest, Hersteller Fa. Biokit, Barcelona). Mit Hilfe eines Antigenverdünnungspuffers wurde eine Antigengebrauchsverdünnung für den Testansatz (Durchführung siehe unten unter 2.) von 1 - 2 x 10⁷ Toxoplasmen/ml eingestellt. Bei einer Ausgangskonzentration von z.B. 10⁸ Toxoplasmen/ml im DA-Reagenz der Fa. LD Labordiagnostika Heiden (Antigenkonzentrat) ergab sich bei einer Mischung von 1 Teil Antigenkonzentrat zu 6 Teilen Antigenverdünnungspuffer ein optimales Sedimentationsbild bei Verwendung von beschichteten Testmodulen. Durch Vorversuche wurde die für das eingesetzte Antigen optimale Gebrauchskonzentration identifiziert. Als Antigenverdünnungspuffer wurde ein Safranin-Borat-Puffer (pH 8,7) mit folgender Zusammensetzung benutzt: 15,45 g Borsäure (F. Merck), 35,1 g NaCI (Fa. Merck), 5 g Na-Azid (Fa. Merck), 20 g bovines Serumalbumin (Fa. Fluka), 0,225 g Safranin (Fa. Merck) in 5000 ml A. bidest., mit 5 n NaOH (Fa. Merck) auf pH 8,7 eingestellt.

### 1.3. Serumverdünnungspuffer

Als Serumverdünnungspuffer wurde ein Bromthymolblau-Borat-Puffer, pH 9,0, folgender Zusammensetzung benutzt: 15.45 g Borsäure (Fa. Merck), 35,1 g NaCI (Fa. Merck), 5 g Na-Azid (Fa. Merck), 20 g bovines Serumalbumin (Fa. Fluka), 0,12g Bromthymolblau (Fa. Merck) in 5000 ml A bidest., mit 5 n NaOH (Fa. Merck) auf pH 9,0 eingestellt. Vor Verwendung wurde durch Zugabe von 0,042 g Dithiothreitol (DTT, Nr. 20710, Fa. Serva, Heidelberg) zu 5 ml Serumverdünnungspuffer der gebrauchsfertige, reduzierende Serumverdünnungspuffer hergestellt.

### 2. Testdurchführung

Alle Testkomponenten wurden vor Testdurchführung auf Raumtemperatur gebracht. 0,042 g DTT wurden in 5 ml Serumverdünnungspuffer gelöst (= gebrauchsfertiger Serumverdünnungspuffer). Nicht benötigter gebrauchsfertiger reduzierender Serumverdünnungspuffer konnte bei -20° C für mindestens 2 Monate eingefroren werden. Bei jedem Testansatz wurden mindestens ein positiv und ein negativ reagierendes Serum als Kontrolle mitgeführt. Entsprechend der benötigten Menge an Antigengebrauchssuspension wurde 1 Teil DA-Antigen-Konzentrat der Fa. LD Labordiagnostika Heiden mit einer durch Vorversuche bestimmten Menge (z.B. mit 6 Volumenteilen) Antigenverdünnungspuffer verdünnt und gut gemischt. Nicht benötigte Antigengebrauchssuspension war bei 4 - 8° C gelagert mindestens 2 Tage verwendbar.
Patientenseren und Kontrollseren wurden als Einfach-Bestimmung angesetzt. Trübe, bakteriell kontaminierte oder hämolytische Seren sollten nicht in diesem Test untersucht werden. Im Einzelnen wurde wie folgt vorgegangen: Pro benötigter Kavität wurden 50 µl gebrauchsfertiger reduzierender Serumverdünnungspuffer vorgelegt. Anschließend wurden je 5 µl Patientenserum bzw. Kontrollserum pro Kavität pipettiert (Serumverdünnung 1 : 11). Dann wurden pro Kavität 50 µl gebrauchsfertige Antigenverdünnung pipettiert (Serumverdünnung 1 : 21). Die Mikrotiterplatte wurde anschließend mit einem Deckel abgedeckt (keine Selbstklebefolie) und ca. 10 Sekunden auf einem Schüttelgerät für Mikrotiterplatten kräftig geschüttelt. Die so behandelten Mikrotiterplatten wurden bei RT (25°C) erschütterungsfrei inkubiert. Die erste Ablesung erfolgte bereits nach 4 - 5 Stunden, eine zweite Ablesung nach Inkubation über Nacht (18 - 24) Stunden) wurde immer durchgeführt.

Spezifisches IgG des Patientenserums wurde bei Bedarf (z.B. Titerverlaufskontrolle) durch eine Titerbestimmung quantifiziert. Das Serum ist dafür in der Regel in 6 Titerstufen (Titrationsfaktor 11) eingesetzt worden, z.B.: 1 : 22, 1 : 242, 1 : 2662, 1 : ca. 30.000, 1: ca. 320.000, 1 : ca. 3,5 Mio.
Die Serumtitration wurde entsprechend dem Screeningansatz in reduzierenden Serumverdünnungspuffer durchgeführt, die übrigen Schritte entsprachen dem oben beschriebenen Vorgehen.
Für die Auswertung günstig war ein vergrößernder Ablesespiegel und ein gleichmäßig heller Hintergrund. Als eindeutig positives Ergebnis wurde nur ein netzartiges Agglutinationsbild ohne Sedimentationsknopf bzw. ohne Sedimentationsring bewertet. Die Auswertung erfolgte stets durch den Vergleich mit den Reaktionsbildern der Kontrollen.

Die nachstehende Tabelle I zeigt die typischerweise erhaltenen Ergebnisse und es wird dokumentiert, daß das erfindungsgemäße Verfahren (DIFA) im Regelfall um den Faktor 10 bis 10.000 empfindlicher als die mit dem indentischen Antigen durchgeführte Direktagglutination (DA) ist und im Vergleich zum Immunfluoreszenz-Test (IFT) nochmals bis um den Faktor 100, also insgesamt bis zum Faktor 10⁵, empfindlicher ist. Diese Empfindlichkeitssteigerung war zu keiner Zeit mit einer verringerten Spezifität verknüpft.
Die Durchführung gemäß vorliegender Erfindung (DIFA) entsprach dem oben geschilderten Verfahren. Die Direktagglutination (DA) wurde völlig analog zum DIFA durchgeführt, jedoch kamen anstelle der erfindungsgemäß beschichteten Module unbehandelte Testmodule zur Anwendung. Die Testdurchführung beim Immunfluoreszenz-Test (IFT) entsprach den Empfehlungen des Bundesgesundheitsamt (BGA), die Objektträger wurden von der Fa. BioMerieux (Nürtingen), die Konjugate von der Fa. Dako, Hamburg, bezogen, verwendet wurde ein übliches Standardserum. Die Serumproben (Probenbez.) stammen aus der Diagnostik des Landesgesundheitsamtes Baden-Württemberg bzw. das Referenzserum aus dem Parasitologischen Institut der Universität Bonn.

**Tabelle I**

| Probenbez. | IFT-Titer | DIFA-Titer | DIFA-Prozone | DA-Titer | DA-Prozone (→= P. bis) |
|---|---|---|---|---|---|
| | | | | | |

| Hohe und mittlere Toxoplasma-spezifische Antikörperkonzentration: | | | | | |
|---|---|---|---|---|---|
| 193 | 16000 | 4,2 x 10⁸ | | 2,9 x 10⁴ | 2662 |
| 157 | 4000 | 4,2 x 10⁸ | | ≤ 2,9 x 10⁴ | → 242 |
| ST 81 | 4000 | 3,9 x 10⁷ | | 3 x 10⁵ | → 242 |
| Ha | 256 | 2,9 x 10⁴ | | 2,9 x 10⁴ | → 2662 |
| Nö | 256 | 3 x 10⁵ | | 2,9 x 10⁴ | → 22 |
| 108 | 256 | 3 x 10⁵ | | 2,9 x 10⁴ | → 2662 |
| Kü | 256 | 3 x 10⁵ | | 2662 | → 2662 |

| Niedrige Toxoplasma-spezifische Antikörperkonzentration: | | | | | |
|---|---|---|---|---|---|
| 3510 | 16 | 2662 | | (242 +/-) | |
| 4171 | 16 | 22 | | (22 + /-) | |
| 4825 | 4 | 22 | | < 22 | |
| 4363 | 32 | 242 | | 22 | |
| 4778 | 16 | 22 | | < 22 | |
| Ba | 64 | 242 | | 22 | |

| Keine Toxoplasma-spezifischen Antikörper nachweisbar: | | | | | |
|---|---|---|---|---|---|
| 4744 | < 2 | < 22 | | < 22 | |
| 3561 | < 2 | < 22 | | < 22 | |
| 3714 | < 16 | < 22 | | < 22 | |
| Na | < 16 | < 22 | | < 22 | |

## Patentansprüche

1. Immunologisches Verfahren auf Basis einer Agglutinationsreaktion, **dadurch gekennzeichnet,** daß eine flüssige immunologisch aktives Material enthaltende Untersuchungsprobe biologischer oder nicht-biologischer Art in Kontakt gebracht wird mit auf einer festen Phase fixierten Antigenen und an der selben festen Phase fixierten Antikörpern und daß die Agglutinationsreaktion ohne einen vorherigen Waschschritt erfolgt.

2. Verfahren nach Anspruch 1, **dadurch gekennzeichnet**, daß die flüssige immunologisch aktives Material enthaltende Untersuchungsprobe biologischer oder nicht-biologischer Art eine unlösliche Antigenpräparation ist.

3. Verfahren nach Anspruch 1, **dadurch gekennzeichnet**, daß die flüssige immunologisch aktives Material enthaltende Untersuchungsprobe biologischer oder nicht-biologischer Art eine unlösliche Antikörperpräparation ist.

4. Verfahren nach Ansprüchen 2 und 3, **dadurch gekennzeichnet,** daß die unlösliche Antigen- oder Antikörperpräparation in korpuskulärer Form vorliegt.

5. Verfahren nach Anspruch 4, **dadurch gekennzeichnet**, daß die in korpuskulärer Form vorliegende Antigen- oder Antikörperpräparation an inerte Partikel gebunden oder adsorbiert ist.

6. Verfahren nach Anspruch 2, **dadurch gekennzeichnet,** daß die unlösliche Antigenpräparation durch chemische Behandlung von a priori löslichen Antigenen nachträglich unlöslich gemacht wurden.

7. Verfahren nach Anspruch 6, **dadurch gekennzeichnet,** daß die unlösliche Antigenpräparation in polymerisierter Form vorliegt.

8. Verfahren nach Anspruch 5, **dadurch gekennzeichnet**, daß die inerten Partikel ausgewählt sind aus der Gruppe natürlicher, synthetischer, organischer und anorganischer Partikel.

9. Verfahren nach Anspruch 8, **dadurch gekennzeichnet**, daß diese Partikel ausgewählt sind aus Blutzellen, Mikroorganismen, Latex und Derivate davon, Bentonit, Kohlenhydrate, Glasperlen, Styrole und Derivate davon, Liposomen, Metalloxidpartikel, Holzkohlepartikel, Gelatine und Derivate davon.

10. Verfahren nach Ansprüchen 1 bis 9 **dadurch gekennzeichnet,** daß das immunologisch aktive Material ein Antikörper der IgX-Klasse einschließlich seiner Unterklassen oder ein Derivat davon, worin X die Bedeutung von G, M, A, D und E hat, oder ein Antigen ist.

11. Verfahren nach Anspruch 10, **dadurch gekennzeichnet**, daß die Antigene und Antikörper ausgewählt sind aus der Gruppe der Proteine, Polypeptide, Oligopeptide, Immunglobuline, Hormone, Proteohormone, Prostaglandine, Enzyme, Tumormarker und Tumorantigene, Mikroorganismen, Pilze, Viren, Protozoen, Drogen, Medikamente und Metaboliten davon, Gifte und gegen diese Stoffe, Organismen und Zellen gebildete Antikörper.

12. Verfahren nach Anspruch 11, **dadurch gekennzeichnet**, daß das Antigen Toxoplasma-spezifisch ist.

13. Verfahren nach einem der Ansprüche 1 bis 12, **dadurch gekennzeichnet,** daß die feste Phase aus einem wasserunlöslichen Material besteht, welches Proteine und/oder Peptide binden oder adsorbieren kann.

14. Verfahren nach Anspruch 15, **dadurch gekennzeichnet,** daß das wasserunlösliche Material aus einem transparenten Material besteht.

15. Verfahren nach Anspruch 14, **dadurch gekennzeichnet**, daß das transparente Material aus Kunstoff, Glas oder einem Mineral oder einem Gemisch davon besteht.

16. Verfahren zur qualitativen und quantiativen Erfassung und/oder diagnostischen Bestimmung eines immunologisch aktiven Materials auf Basis einer Agglutinationsreaktion **gekennzeichnet durch** die Schritte
a) Beschichten einer festen Phase mit einem Antikörper und zusätzlich einem Antigen, welche mit einem in einer Untersuchungsprobe biologischer oder nicht-biologischer Art enthaltendem immunologisch aktiven Material reagiert,
b) Blockieren der unter a) erhaltenen festen Phase mit einem geeigneten Blockierungsmittel, welches hinsichtlich der Herkunft des in der Untersuchungsprobe enthaltenden immunologisch aktiven Materials aus nicht verwandten Fremdproteinen besteht,
c) In-Kontakt-bringen der nach Schritt b) hergestellten festen Phase mit einer Untersuchungsprobe biologischer oder nicht-biologischer Art enthaltend immunologisch aktives Material,
d) Bestimmen, Ablesen oder Dokumentieren der eingetretenen Agglutination.

17. Verfahren nach Anspruch 16, **dadurch gekennzeichnet**, daß das die Untersuchungsprobe biologischer oder nicht-biologischer Art enthaltende immunologisch aktives Material gemäß Ansprüchen 2 bis 12 definiert ist und die feste Phase aus einem Material gemäß Ansprüchen 13 bis 15 besteht.

18. Formkörper bestehend aus einem wasserunlöslichen Material, **dadurch gekennzeichnet**, daß an seiner Oberfläche ein Antikörper und ein Antigen vor einer nachfolgenden Agglutinationsreaktion immobilisert bzw. gecoatet ist und woran die verbliebene freie Kapazität der selben Oberfläche durch ein Blockierungsmittel blockiert ist.

19. Formkörper nach Anspruch 18, **dadurch gekennzeichnet**, daß das Blockierungsmittel hinsichtlich des besagten Antikörpers und Antigens aus einem nicht verwandten Fremdprotein besteht

20. Formkörper nach Anspruch 19, **dadurch gekennzeichnet,** daß das Blockierungsmittel Serumalbumin oder Gelatine ist.

21. Formkörper nach Ansprüchen 18 bis 20, **dadurch gekennzeichnet,** daß dieser aus an einem Ende verschlossenen Hohlkörper besteht oder daß er flächig ausgestaltet ist und in dieser Ausgestaltung mindestens eine Vertiefung oder Mulde aufweist.

22. Formkörper nach einem der Ansprüche 18 bis 21, **dadurch gekennzeichnet**, daß die daran immobilisierten bzw. gecoateten Antikörper monovalente, bivalente, polyvalente, polyklonale oder monoklonale Antikörper und Fragmente und Derivate davon umfassen.

23. Formkörper nach einem der Ansprüche 18 bis 22, **dadurch gekennzeichnet**, daß die daran immobilisierten bzw. gecoateten Antigene Proteine, Polypeptide, Oligopeptide, Immunglobuline, Hormone, Proteohormone, Prostaglandine, Enzyme, Tumormarker und Tumorantigene, Mikroorganismen, Pilze, Viren, Protozoen, Drogen, Medikamente und Metaboliten davon, Gifte und gegen diese Stoffe, Organismen und Zellen gebildete Antikörper umfassen.

24. Verwendung eines Formkörpers nach Ansprüchen 18 bis 23 für ein immunologisches Verfahren nach Ansprüchen 1 bis 17.

25. Kit für die qualitative und quantitative Erfassung und/oder diagnostische Bestimmung eines immunologisch aktiven Materials auf Basis einer Agglutinationsreaktion, **dadurch gekennzeichnet,** daß dieser aus den Komponenten
a) Formkörper nach einem der Ansprüche 18 bis 23,
b) immunologisch aktives Material bestehend aus einer geeigneten Antigenpräparation und/oder einer geeigneten Antikörperpräparation nach einem der Ansprüche 2 bis 12,
und gegebenenfalls
c) ein Verdünnungspuffer für das immunologisch aktive Material,
d) ein positives und/oder negatives Kontrollserum bzw. eine Antigenkontrolle,
besteht.

26. Kit nach Anspruch 25, **dadurch gekennzeichnet**, daß der Verdünnungspuffer ein Borat-Puffer ist und ein Reduktionsmittel enthält.

27. Kit nach Anspruch 264, **dadurch gekennzeichnet,** daß das Reduktionsmittel Dithiothreitol ist.

## Claims

1. Immunological method on the basis of an agglutination reaction, characterized in that a liquid test sample of biological or non-biological nature containing immunologically active material is contacted with antigens immobilised on a solid phase and with antibodies immobilised on the same solid phase and that the agglutination reaction occurs without a prior washing step.

2. Method according to claim 1, characterised in that the liquid test sample of biological or non-biological nature containing immunologically active material is an insoluble antigen preparation.

3. Method according to claim 1, characterised in that the liquid test sample of biological or non-biological nature containing immunologically active material is an insoluble antibody preparation.

4. Method according to claims 2 and 3, characterised in that the insoluble antigen preparation or the insoluble antibody preparation are present in corpuscular form.

5. Method according to claim 4, characterised in that the antigen preparation or the antibody preparation which are present in corpuscular form are bound to or adsorbed on inert particles.

6. Method according to claim 2, characterised in that the insoluble antigen preparation was subsequently made insoluble by chemical treatment of *a priori* soluble antigens.

7. Method according to claim 6, characterised in that the insoluble antigen preparation is present in polymerised form.

8. Method according to claim 5, characterised in that the inert particles are selected from the group of natural, synthetic, organic and inorganic particles.

9. Method according to claim 8, characterised in that the particles are selected from the group consisting of blood cells, microorganisms, latex and derivatives thereof, bentonite, carbohydrates, glass beads, styroles and derivatives thereof, liposomes, metal oxide particles, charcoal particles, gelatine and derivatives thereof.

10. Method according to claims 1 to 9, characterised in that the immunologically active material is an antibody of the IgX class including its subclasses or a derivative thereof, wherein X is G, M, A, D and E, or an antigen.

11. Method according to claim 10, characterised in that the antigens and antibodies are selected from the group consisting of proteins, polypeptides, oligopeptides, immunoglobulines, hormones, proteohormones, prostaglandines, enzymes, tumor markers and tumor antigens, microorganisms, fungi. viruses, protozoa, drugs, medicaments and metabolites thereof, toxins and antibodies produced against these substances, organisms and cells.

12. Method according to claim 11, characterised in that the antigen is toxoplasma-specific.

13. Method according to one of claims 1 to 12, characterised in that the solid phase consists of a material insoluble in water capable of binding or adsorbing proteins and/or peptides.

14. Method according to claim 13, characterised in that the material insoluble in water consists of a transparent material.

15. Method according to claim 14, characterised in that the transparent material consists of synthetic material, glass or a mineral or a mixture thereof.

16. Method for the qualitative and quantitative detection and/or diagnostic determination of an immunologically active material on the basis of an agglutination reaction, characterised in by the steps
a) coating a solid phase with an antibody and, additionally, with an antigen, which react with an immunologically active material contained in a test sample of biological or non-biological nature,
b) blocking the solid phase obtained by step a) with a suitable blocking agent which, in respect of the origin of the immunologically active material contained in the test sample, consists of non-related foreign proteins,
c) contacting the solid phase produced by step b) with a test sample of biological or non-biological nature containing immunologically active material,
d) determining, reading or documenting the occuring agglutination.

17. Method according to claim 16, characterised in that the immunologically active material contained in a test sample of biological or non-biological nature is defined according to claims 2 to 12 and that the solid phase consists of a material according to claims 13 to 15.

18. Moulding consisting of a material insoluble in water, characterised in that an antibody and an antigen is immobilised or coated on the surface of said moulding prior a subsequent agglutination reaction and on which the remaining free capacity of the same surface is blocked by a blockling agent.

19. Moulding according to claim 18, characterised in that, in respect of said antibody and said antigen, the blocking agent consists of a non-related foreign protein.

20. Moulding according to claim 19, characterised in that the blocking agent is serum albumine or gelatine.

21. Moulding according to claims 18 to 20, characterised in that it consists of a hollow body closed at one end or that it is flat-shaped with at least one cavity or trough.

22. Moulding according to one of claims 18 to 21, characterised in that the antibodies immobilised or coated on it comprise monovalent, bivalent, polyvalent, polyclonal or monoclonal antibodies and fragments or derivatives thereof.

23. Moulding according to one of claims 18 to 22, characterised in that the antigens immobilised or coated on it comprise proteins, polypeptides, oligopeptides, immunoglobulines, hormones, proteohormones, prostaglandines, enzymes, tumor markers and tumor antigens, microorganisms, fungi, viruses, protozoa, drugs, medicaments and metabolites thereof, toxins and antibodies produced against these substances, organisms and cells.

24. Use of a moulding according to claims 18 to 23 for an immunological method according to claims 1 to 17.

25. Kit for the qualitative and quantitative detection and/or diagnostic determination of an immunologically active material on the basis of an agglutination reaction, characterised in that it consists of the components
a) moulding according to one of claims 18 to 23,
b) immunologically active material consisting of a suitable antigen preparation and/or a suitable antibody preparation according to claims 2 to 12,
c) a dilution buffer for the immunologically active material,
d) a positive and/or negative control serum or an antigen control, respectively.

26. Kit according to claim 25, characterised in that the dilution buffer is a borate buffer that contains a reducing agent.

27. Kit according to claim 26, characterised in that the reducing agent is dithiothreitol.

## Revendications

1. Procédé immunologique à base d'une réaction d'agglutination, caractérisé en ce qu'un échantillon d'examen de type biologique ou non biologique liquide contenant une matière immunologiquement active est mis en contact avec des antigènes fixés sur une phase solide et avec des anticorps fixés sur la même phase solide et en ce que la réaction d'agglutination a lieu sans étape de lavage préalable.

2. Procédé selon la revendication 1 caractérisé en ce que l'échantillon d'examen de type biologique ou non biologique liquide contenant une matière immunologiquement active est une préparation d'antigènes insoluble.

3. Procédé selon la revendication 1 caractérisé en ce que l'échantillon d'examen de type biologique ou non biologique liquide contenant une matière immunologiquement active est une préparation d'anticorps insoluble.

4. Procédé selon les revendications 2 et 3 caractérisé en ce que la préparation d'antigènes ou d'anticorps insoluble est sous forme corpusculaire.

5. Procédé selon la revendication 4 caractérisé en ce que la préparation d'antigènes ou d'anticorps sous forme corpusculaire est fixée ou adsorbée sur des particules inertes.

6. Procédé selon la revendication 2 caractérisé en ce que la préparation d'antigènes insoluble a été rendue insoluble ultérieurement par traitement chimique d'antigènes a priori solubles.

7. Procédé selon la revendication 6 caractérisé en ce que la préparation d'antigènes insoluble est sous forme polymérisée.

8. Procédé selon la revendication 5 caractérisé en ce que les particules inertes sont choisies dans le groupe des particules naturelles, synthétiques, organiques et inorganiques.

9. Procédé selon la revendication 8 caractérisé en ce que ces particules sont choisies parmi les cellules sanguines, les micro-organismes, les latex et leurs dérivés, la bentonite, les glucides, les billes de verre, les styrènes et leurs dérivés, les liposomes, les particules d'oxydes métalliques, les particules de charbon de bois, la gélatine et leurs dérivés.

10. Procédé selon les revendications 1 à 9 caractérisé en ce que la matière immunologiquement active est un anticorps de la classe IgX y compris ses sous-classes ou un dérivé de celui-ci, X ayant la signification de G, M, A, D et E, ou un antigène.

11. Procédé selon la revendication 10 caractérisé en ce que les antigènes et les anticorps sont choisis dans le groupe des protéines, des polypeptides, des oligopeptides, des immunoglobulines, des hormones, des protéohormones, des prostaglandines, des enzymes, des marqueurs tumoraux et des antigènes tumoraux, des micro-organismes, des champignons, des virus, des protozoaires, des drogues, des médicaments et de leurs métabolites, des poisons et des anticorps formés contre ces substances, ces organismes et ces cellules.

12. Procédé selon la revendication 11 caractérisé en ce que l'antigène est spécifique de toxoplasmes.

13. Procédé selon l'une des revendications 1 à 12 caractérisé en ce que la phase solide consiste en une matière insoluble dans l'eau qui peut fixer ou adsorber des protéines et/ou des peptides.

14. Procédé selon la revendication 13 caractérisé en ce que la matière insoluble dans l'eau consiste en une matière transparente.

15. Procédé selon la revendication 14 caractérisé en ce que la matière transparente consiste en matière plastique, en verre ou en un minéral, ou en un mélange de ceux-ci.

16. Procédé pour la détection qualitative et quantitative et/ou pour la détermination diagnostique d'une matière immunologiquement active à base d'une réaction d'agglutination caractérisé par les étapes suivantes
a) recouvrement d'une phase solide avec un anticorps et en outre un antigène, qui réagissent avec une matière immunologiquement active contenue dans un échantillon d'examen de type biologique ou non biologique,
b) blocage de la phase solide obtenue en a) avec un agent de blocage approprié qui consiste en des protéines étrangères non apparentées concernant l'origine de la matière immunologiquement active contenue dans l'échantillon d'examen,
c) mise en contact de la phase solide préparée selon l'étape b) avec un échantillon d'examen de type biologique ou non biologique contenant une matière immunologiquement active,
d) détermination, lecture ou vérification de l'agglutination apparue.

17. Procédé selon la revendication 16 caractérisé en ce que la matière immunologiquement active contenant l'échantillon d'examen de type biologique ou non biologique est définie selon les revendications 2 à 12 et la phase solide consiste en une matière selon les revendications 13 à 15.

18. Corps façonné consistant en une matière insoluble dans l'eau caractérisé en ce qu'un anticorps et un antigène sont immobilisés ou fixés sur sa surface avant une réaction d'agglutination consécutive et sur lequel la capacité libre restante de la même surface est bloquée par un agent de blocage.

19. Corps façonné selon la revendication 18 caractérisé en ce que l'agent de blocage consiste en une protéine étrangère non apparentée concernant ledit anticorps et ledit antigène.

20. Corps façonné selon la revendication 19 caractérisé en ce que l'agent de blocage est la sérumalbumine ou la gélatine.

21. Corps façonné selon les revendications 18 à 20 caractérisé en ce que celui-ci consiste en corps creux fermés à une extrémité ou en ce qu'il est plan et comporte, dans cette configuration, au moins un évidemment ou cavité.

22. Corps façonné selon l'une des revendications 18 à 21 caractérisé en ce que les anticorps immobilisés ou fixés sur lui comprennent des anticorps polyclonaux ou monoclonaux monovalents, divalents, multivalents et leurs fragments et dérivés.

23. Corps façonné selon l'une des revendications 18 à 22 caractérisé en ce que les antigènes immobilisés ou fixés sur lui comprennent des protéines, des polypeptides, des oligopeptides, des immunoglobulines, des hormones, des protéohormones, des prostaglandines, des enzymes, des marqueurs tumoraux et des antigènes tumoraux, des micro-organismes, des champignons, des virus, des protozoaires, des drogues, des médicaments et leurs métabolites, des poisons et des anticorps formés contre ces substances, ces organismes et ces cellules.

24. Utilisation d'un corps façonné selon les revendications 18 à 23 pour un procédé immunologique selon les revendications 1 à 17.

25. Trousse pour la détection qualitative et quantitative et/ou la détermination diagnostique d'une matière immunclogiquement active à base d'une réaction d'agglutination, caractérisée en ce qu'elle consiste en les composants
a) corps façonné selon l'une des revendications 18 à 23,
b) matière immunologiquement active consistant en une préparation d'antigènes appropriée et/ou une préparation d'anticorps appropriée selon l'une des revendications 2 à 12,
et éventuellement
c) un tampon de dilution pour la matière immunologiquement active,
d) un sérum témoin positif et/ou négatif ou un témoin antigénique.

26. Trousse selon la revendication 25 caractérisée en ce que le tampon de dilution est un tampon borate et contient un réducteur.

27. trousse selon la revendication 26 caractérisée en ce que le réducteur est le dithiothréitol.
